# FASCICULE DE BREVET EUROPEEN

(11) **EP 1 066 405 B1**
(45) Date de publication et mention de la délivrance du brevet: **25.10.2006**
(21) Numéro de dépôt: 99911874.8
(22) Date de dépôt: 02.04.1999
(51) Int. Cl.: C12Q 1/34, C12Q 1/04

(54) **NOUVEAUX SUBSTRATS CHROMOGENES POUR LA DETECTION D'HYDROLASES BACTERIENNES**
NEUE CHROMOGENE SUBSTRATE ZUM NACHWEIS VON BAKTERIELLEN HYDROLASEN
NOVEL CHROMOGENOUS SUBSTRATES FOR DETECTING BACTERIAL HYDROLASE

(30) Priorité: 02.04.1998 FR 9804332
(43) Date de publication de la demande: 10.01.2001
(73) Titulaire: BIO MERIEUX, 69280 Marcy l'Etoile (FR)
(72) Inventeur: ARMSTRONG, Lyle, Ashington, Northumberland NE63 8AE (GB); JAMES, Arthur, Newcastle upon Tyne NE2 1HR (GB); MONGET, Daniel, F-01150 Saint-Sorlin-en-Bugey (FR); ORENGA, Sylvain, F-01160 Neuville-sur-Ain (FR)
(74) Mandataire: Guerre, Dominique
(86) Numéro de dépôt international: PCT/FR1999/000781
(87) Numéro de publication internationale: WO 1999/051767

(56) Documents cités:
- EP-A- 0 053 470
- EP-A- 0 138 530
- EP-A- 0 150 227
- DE-A- 4 324 392
- FR-A- 2 708 286
- US-A- 4 588 836
- US-A- 4 603 108
- US-A- 4 637 979
- US-A- 4 681 841
- J F CORBETT: "Benzoquinone Imines. Part VIII. Mechanism and Kinetics of the Reaction of p-Benzoquinone Monoimines with Monohydric Phenols." JOURNAL OF THE CHEMICAL SOCIETY B,1970, pages 1502-1509, XP002087428

## Description

La présente invention concerne la mise en évidence d'enzymes de type hydrolases, en particulier de peptidases, par l'utilisation de substrats chromogènes efficaces. La présente invention concerne également un procédé et un dispositif d'identification de micro-organismes qui sont à la fois simples et fiables.

Depuis de très nombreuses années, on utilise des substrats particuliers pour permettre la détermination de la présence ou de l'absence d'activités enzymatiques caractéristiques de bactéries. Par le choix des substrats, selon qu'il y a réaction ou non, il est possible de caractériser la nature d'un genre de bactéries ou de différencier des espèces d'un genre bactérien donné.

Les substrats synthétiques d'enzymes sont constitués de deux parties, une première partie spécifique de l'activité enzymatique à révéler, et une seconde partie faisant office de marqueur, ci-après appelée partie marqueur.

Ces substrats particuliers peuvent être des substrats fluorescents ou chromogènes. En fait, c'est la seconde partie ou partie marqueur qui est fluorescente ou chromogène, lorsqu'elle n'est pas associée à la première partie.

Les substrats fluorescents peuvent être de différentes compositions.

Tout d'abord les substrats à base d'umbelliférone ou d'aminocoumarine, et ses dérivés substitués en position 2, qui permettent la libération d'un composé fluorescent de couleur variant du bleu au vert sous lampe à ultraviolets (UV) (λₑₓ= 365 nm).

Ensuite les substrats à base de résorufine (et dérivés) où il y a libération d'un composé fluorescent rose sous lumière naturelle (λₑₓ= 530 nm).

Enfin les substrats à base de fluorescéïne (et dérivés) qui, après dégradation, libère un composé fluorescent jaune sous lumière naturelle (λₑₓ= 485 nm).

Ces substrats sont peu adaptés à une utilisation dans des milieux gélosés, et sont plus utilisés en milieu liquide.

Les substrats chromogènes peuvent également être de différentes natures.

Premièrement, il y a les substrats à base d'indoxyl et ses dérivés qui, en présence d'oxygène, produisent un précipité variant du bleu au rose.

Leurs applications sont essentiellement limitées aux osidases, estérases et ne concernent pas la détection d'une activité peptidase. Bien adaptés à une utilisation sur support solide (filtre, gélose, gel d'électrophorèse, ...), ils sont par contre moins bien adaptés à une utilisation en milieu aqueux liquide (formation d'un précipité).

Deuxièmement, il y a les substrats à base d'hydroxyquinoline ou d'esculétine et leurs dérivés, qui en présence de sels de fer, produisent un précipité brun.

Encore une fois leurs applications sont limitées aux osidases et estérases. Ils sont adaptés à une utilisation sur support solide, et peu adaptés à une utilisation en milieu aqueux liquide.

Troisièmement, il existe des substrats à base de nitrophénol et nitroaniline et dérivés, où l'on obtient la formation d'un composé jaune.

Ils permettent de détecter les activités osidases et estérases dans le cas de substrats à base de nitrophénol, et des activités peptidases dans le cas de substrats à base de nitroaniline. Cependant, dans le cas de la détection d'activités peptidases, la nitroaniline libérée est toxique pour les bactéries que l'on souhaite identifier ou caractériser, ce qui peut être préjudiciable pour des analyses en cours ou ultérieures. D'autre part, ils sont peu adaptés à une utilisation sur support solide, et mieux adaptés à une utilisation en milieu liquide. De plus, ils sont peu chromogènes du fait du coefficient d'extinction relativement faible de la couleur (jaune) dont le contraste est peu prononcé dans les milieux biologiques.

Quatrièmement, il y a les substrats à base de naphtol et naphtylamine et ses dérivés. Dans ce cas, la réaction s'effectue en deux temps, le naphtol ou naphtylamine libéré par l'activité enzymatique subit un "azo-coupling" en présence d'un sel de diazonium qui est ajouté au moment de la révélation, conduisant à la formation d'un composé insoluble coloré.

Ils permettent de détecter les activités osidases et estérases par l'intermédiaire du naphtol, et les activités peptidases par l'intermédiaire de la naphtylamine. La réaction d'"azo-coupling" s'effectue dans un milieu souvent chimiquement agressif, toxique pour les bactéries et rendant l'échantillon inutilisable pour d'autres analyses, de plus les naphtylamines sont cancérigènes.

Pour révéler la naphtylamine et donc une activité peptidase, il est aussi possible d'ajouter en fin de réaction enzymatique du p-diméthylaminocinnamaldehyde en milieu acide, à la place d'un sel de diazonium, avec toujours des inconvénients de toxicité vis-à-vis de l'échantillon analysé.

La demande de brevet FR-A-2.708.286 propose d'utiliser un mélange de substrats chromogènes, chaque chromogène fournit une coloration particulière pour une enzyme spécifique qui est différente de la coloration et de l'enzyme associées à l'autre chromogène. Lorsque les deux colorations et donc les deux enzymes sont présentes, il y a formation d'une « tierce coloration ».

Cette technique n'est toutefois pas satisfaisante car en fonction de la faible concentration de l'une des deux enzymes, il n'est pas possible de détecter cette activité enzymatique qui est alors masquée par la coloration associée à l'enzyme dont la concentration est prépondérante.

Enfin, les brevets US-A-4,681,841 et US-A-4,588,836 décrivent une méthode indirecte de détection d'une seule activité enzymatique utilisant un couplage entre un aminobenzène et un dérivé aromatique hydroxylé (par exemple l'alpha-naphtol), couplage qui engendre la formation d'un indicateur chromogène en présence d'oxydase. L'un des deux composés (l'aminobenzène) entre dans la composition du substrat de départ : si l'activité enzymatique recherchée est présente, ce composé sera libéré et pourra réagir avec l'autre composé.

Néanmoins, ces documents ne permettent pas de déduire qu'il est possible de détecter au moins deux activités enzymatiques par l'intermédiaire d'au moins deux molécules non chromogènes. Seule la présence de ces au moins deux activités génère ainsi une molécule chromogène. L'intérêt d'une telle technique est pertinent lorsque la bactérie à identifier comporte au moins une activité enzymatique en commun avec d'autres bactéries et au moins une activité enzymatique différente pour ces mêmes autres bactéries. De plus, l'intérêt de cette technique est particulièrement pertinent lorsque la bactérie à identifier comporte au moins une activité enzymatique en commun avec d'autres bactéries et au moins une activité enzymatique différente pour encore d'autres bactéries, la bactérie à identifier étant la seule à avoir ces au moins deux activités enzymatiques.

Il est donc aisé de comprendre qu'il n'existe pas aujourd'hui de substrat non chromogène, qui permet de révéler au moins deux activités enzymatiques qui soit particulièrement efficace et intéressant.

Concernant le procédé et le dispositif d'identification, l'état de la technique est constitué par un procédé d'identification qui prévoit une manipulation en trois étapes:
- prélèvement de la colonie à identifier,
- réalisation d'un test d'orientation, et
- recherche de colonies semblables à celles observées et réalisation d'un inoculum.
Le test d'orientation doit être pratiqué avant l'utilisation d'un système d'identification. C'est notamment le cas pour la coloration de Gram qui nécessite une observation microscopique.

Cependant, cette coloration n'est pas toujours facile à réaliser et surtout à interpréter. D'autre part, le coût engendré par ce test est loin d'être négligeable.

L'un des objectifs de la présente invention est donc de créer le lien entre un milieu de culture et les systèmes d'identification et d'antibiogramme en offrant aux biologistes la possibilité d'effectuer un test simple en une étape permettant à la fois la confirmation du résultat de la coloration de Gram et la préparation de l'inoculum. Ainsi le choix des tests d'identification et d'antibiogramme est fiabilisé.

La demande de brevet EP-A-0.122.028 propose une méthode de colorimétrie permettant de détecter la présence d'au moins une enzyme suspectée d'être présente dans un échantillon biologique. Elle préconise de préparer un matériau absorbant monté sur un support rigide, tel qu'un écouvillon. en absorbant dans ce matériau au moins un substrat spécifique de l'enzyme que l'on souhaite détecter. Préalablement à son utilisation, le matériau absorbant contenant le ou les substrats est séché.

L'invention permet la mise en évidence colorimétrique d'activités enzymatiques de type hydrolases (osidases, estérases, phosphatases, peptidases) à l'aide de substrats synthétiques à base de deux composés, ainsi qu'un procédé nouveau qui peut être appliquée à des milieux réactionnels aussi bien liquides que solides.

Elle propose également un procédé et un dispositif d'identification de micro-organismes, qui orientent l'identification, et permettent la récupération desdits micro-organismes pour permettre d'effectuer un inoculum. Cet inoculum permet d'utiliser les mêmes micro-organismes pour une ou plusieurs autres étapes, tels qu'une identification, un antibiogramme.

Après hydrolyse des substrats, la détection des activités enzymatiques est basée sur la formation d'un complexe coloré à partir du couplage oxydatif des deux composés précédemment cités. Ce couplage oxydatif peut être facilité par un oxydant ajouté au milieu réactionnel ou produit lors d'un processus métabolique au sein de ce milieu, ou plus simplement par la présence d'oxygène endogène dans ce même milieu.

Selon une première interprétation, l'invention concerne une association de deux entités distinctes, correspondant chacune à un substrat différent et permettant de détecter la présence de l'activité enzymatique d'au moins deux enzymes. Chaque substrat est caractérisé par le fait qu'il est constitué d'une partie spécifique de l'enzyme recherchée, et d'une molécule non chromogène constituant la partie marqueur du substrat et que les deux molécules non chromogènes correspondant aux deux substrats réagissent ensemble quand elles sont sous forme libre et peuvent générer une molécule chromogène.

Selon une seconde interprétation qui sera plus particulièrement utilisée par la suite, pour arriver à ce résultat, il est nécessaire d'utiliser une nouvelle forme de substrats. Ainsi, le substrat selon l'invention comporte deux molécules différentes. L'une des molécules est constituée d'une partie spécifique, d'une activité enzymatique, et d'une partie marqueur, distincte d'une molécule chromogène. L'autre molécule comporte toujours la partie marqueur, distincte d'une molécule chromogène, qui est associée avec au moins une partie spécifique, d'une activité enzymatique. L'objet de l'invention est donc, si les activités enzymatiques sont présentes, de révéler la formation d'une molécule chromogène qui est efficace uniquement lorsque les deux parties marqueur, une fois libérées, se sont associées.

La présente invention concerne une composition comprenant deux substrats permettant de détecter la présence de l'activité enzymatique d'au moins deux enzymes, caractérisée par le fait, qu'elle est constituée d'au moins deux molécules, une première molécule constituée d'une partie marqueur-non chromogène associée à au moins une partie spécifique cible de la première enzyme et une seconde molécule constituée d'une autre partie marqueur non chromogène associée à une partie spécifique cible de la seconde enzyme, et que les parties marqueur non chromogènes, une fois libérées, réagissent pour former une molécule chromogène.

Quel que soit le mode de réalisation des substrats, la partie marqueur non chromogène de la première molécule est constituée d'aminobenzène ou l'un de ses dérivés : 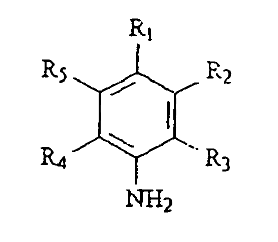 que la partie marqueur non chromogène de la seconde molécule est constituée d'α-naphtol ou l'un de ses dérivés : 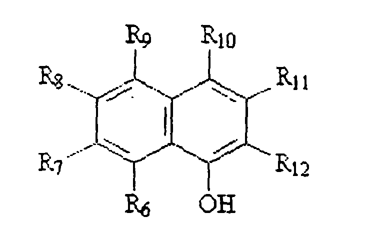 et que la molécule chromogène obtenue est constituée par :

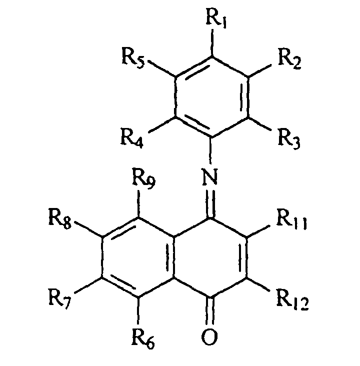

Dans ce cas le radical R1 est constitué de -OH ou -SH ou 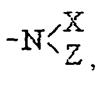 le radical R₁₀ est constitué de -H ou d'un atome, tel que -Br, -Cl, -I, ou un groupe d'atomes, tel que-SH, qui peut être enlevé durant le couplage oxydatif, et chaque radical R₂ à R₉, R₁₁ ou R₁₂ est constitué de -H, -OH, -Br, -Cl, -I, ou autres substituants plus complexes, tels que -CH₃, -CH₂CH₃, -OCH₃-OCH₂CH₃, -COOH.

Dans le cas du radical R₁ X et/ou Z est constitué de -H, ou autres substituants plus complexes, tels que - CH₃, - CH₂CH₃,

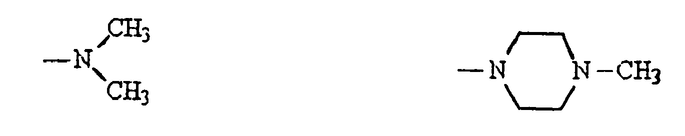

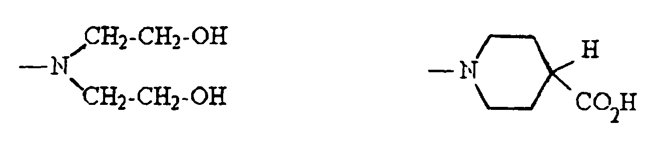

Selon une variante de réalisation, au moins l'un des couples de radicaux R₂/R₃ et R₄/R₅ est constitué d'un système aromatique, alicyclique ou hétérocyclique.

La présente invention concerne encore un procédé de détection d'au moins deux activités enzymatiques par l'intermédiaire de substrats, tels que décrits ci-dessus, qui consiste à :
- mettre les substrats en présence d'au moins un type de bactéries,
- attendre que les bactéries hydrolysent les substrats, et
- détecter les activités enzymatiques basées sur la formation d'un complexe coloré à partir du couplage oxydatif des deux parties marqueur.

Un autre objet de la présente invention est de proposer un procédé, dans lequel les molécules sont présentes dans un matériau absorbant et sont mis en contact avec l'échantillon, et, après détection, les micro-organismes ainsi prélevés sont remis en suspension afin de permettre des analyses ultérieures (identifications, antibiogrammes, etc.).

Les molécules et autres composés entrant dans la composition réactionnelle contenue dans le matériau absorbant sont :
- la première molécule constituée d'amino-benzène ou un de ses dérivés, et
- la seconde molécule constituée d'α-naphtol ou un de ses dérivés.

La composition réactionnelle contient également un oxydant, tel que le ferricyanate de potassium.

Parmi les autres composés, il est possible d'avoir également un activateur de la réaction, tel qu'une faible quantité de la première molécule et/ou de la deuxième molécule, présente(s) dans l'échantillon à tester.

Toujours parmi les autres composés, il est possible que la composition comprenne un liant ou colle, tel que la Poly Vinyl Pyrolidone (PVP).

Le procédé peut être utilisé pour identifier le Gram d'une espèce bactérienne à tester, dans ce cas, la première molécule est constituée par de l'AlaDMpPD, et que la seconde molécule est constituée par de l'α-naphtol.

De plus, la composition du mélange réactionnel absorbé par le matériau absorbant est la suivante :
- α-naphtol de 0,01 à 5 g/l, préférentiellement de 0,1 à 1 g/l, et par exemple de 0,5 g/l.
- ferricyanate de potassium de 0,01 à 5 g/l, préférentiellement de 0,1 à 1 g/l, et par exemple de 0,5 g/l, et
- AlaDMpPD de 0,01 à 5 g/l, préférentiellement de 0,1 à 1 g/l, et par exemple de 0,35 g/l.

Selon une variante, l'activateur est constitué par de- l'Ala-DMpPD à une concentration de 0,01 à 0,5 g/l, préférentiellement de 0,05 à 0,1 g/l, et par exemple de 0,075 g/l.

Selon une autre variante, la composition comprend en outre de 1 à 50 g/l, préférentiellement de 10 à 25 g/l, et par exemple de 15 g/l de PVP.

L'invention concerne aussi l'utilisation de substrats, tels que décrits ci-dessus, pour la détection d'une activité enzymatique de type peptidase.

Enfin, la présente invention a trait à un dispositif d'identification permettant la mise en oeuvre du procédé d'identification décrit ci-dessus, il est constitué par un support, par exemple en plastique, qui est inerte vis-à-vis du matériau absorbant et des substrats qu'il contient et/ou vis-à-vis de l'échantillon testé, sur lequel est monté une tête en matériau absorbant, tel que du viscose, ledit dispositif comportant la composition de l'invention.

### I - Nouveaux substrats chromogènes et procédé d'utilisation :

L'invention, qui sera décrite ci-après, a trait à quelques modes de réalisation particuliers de l'invention. Ces modes de réalisation ne sont donc pas limitatifs de la portée de la présente invention qui peut être utilisée pour la détection de tout type d'activités enzymatiques, de type hydrolase, et pour tout genre ou type de micro-organismes.

La composition comprenant deux substrats, permettant de détecter la présence de l'activité enzymatique d'au moins deux enzymes, est constituée d'au moins deux molécules, une première molécule constituée d'une partie marqueur non chromogène associée à au moins une partie spécifique cible de la première enzyme et une seconde molécule constituée d'une autre partie marqueur non chromogène associée à une partie spécifique cible de la seconde enzyme. Les parties marqueur non chromogènes, une fois libérées, réagissent pour former une molécule chromogène.

Les deux parties marqueur sont respectivement constituées par les composés 1 et Il ci-dessous. 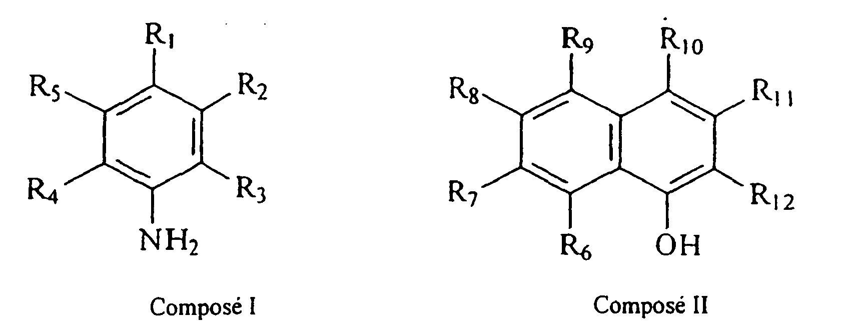 où :
- R1 = -OH ou -SH ou 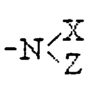 avec X et/ou Z = -H ou autres substituants plus complexes, comme -CH₃, -CH₂CH₃, 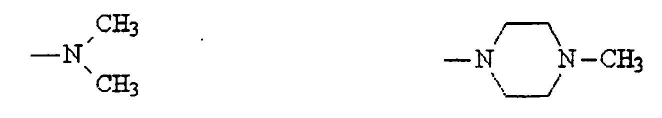 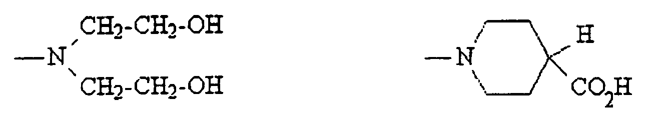
- R₁₀ = -H ou un groupe ou atome qui peut être enlevé durant le couplage oxydatif, comme -Br, -CI, -I, -SH, etc.
- R₂, R₃, R₄, R₅, R₇, R₈, R₉, R₁₁ et R₁₂ = -H, -OH, -Br, -Cl, -I, -CH3,-CH2CH3, -OCH3, -OCH2CH3, -COOH, ou tout autre substituant plus complexe, R₂/R₃ et R₄/R₅ pouvant faire également partie d'un système aromatique, alicyclique ou même hétérocyclique, comme :

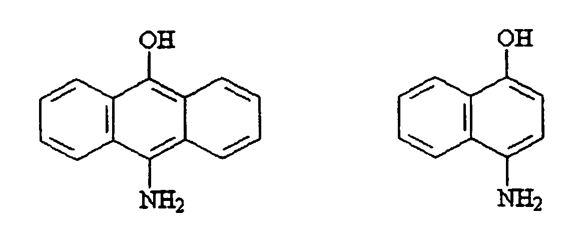

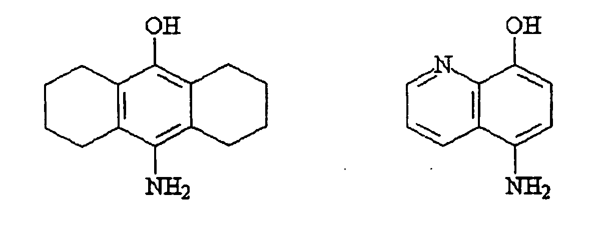

Le composé III obtenu par la combinaison des deux composés I et II est donc le suivant :

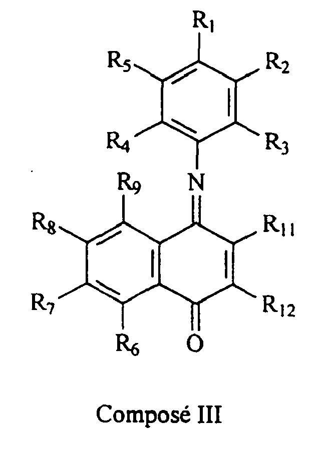

Pour comprendre l'invention, il est rappelé que la formule d'un substrat enzymatique d'hydrolase peut être schématisée sous forme :
- A - O - B, s'il s'agit d'un substrat d'osidase, de phosphatase, ou d'estérase ou de sulfatase.
   L'hydrolyse enzymatique peut alors s'écrire :

   A - O -B + H₂O ⇒ A-OH + B-OH,

   avec selon l'invention :
   A-OH = ose, phosphate, sulfate, acide gras (depuis le plus simple c'est-à-dire l'acide acétique), et
   B-OH = composé I avec R₁ = -OH ou composé II.
- A-CO-NH-B, s'il s'agit d'un substrat de peptidase.
   L'hydrolyse enzymatique peut alors s'écrire :

   A-CO-NH-B + H₂O ⇒ A-COOH + B-NH₂,

   avec selon l'invention :
   A-COOH = acide aminé ou chaîne d'acides aminés terminés ou non par un agent bloquant, et
   B-NH₂ = composé I.

La formation dans le milieu réactionnel du complexe coloré III (dérivé de l'indophénol) à partir du couplage oxydatif de I et II permet de déceler l'hydrolyse du substrat et donc l'activité enzymatique impliquée.

La couleur du composé III dépend des substituants sur les composés I et II.

Elle est par exemple pourpre, si R₁ = -OH et R₂ à R₁₁ = -H, ou bleue, dans le cas où R₁ = -OH, R₂ et R₅ = -Cl et R₃ à R₁₁ = -H.

Le procédé ainsi décrit est en particulier extrêmement intéressant pour déceler colorimétriquement des activités de type peptidases. En effet, selon l'état de la technique antérieur, le choix des réactions colorées se limitait à l'utilisation de substrats à base de nitroaniline, peu chromogène, et de substrats à base de naphtylamine très toxique, nécessitant l'addition d'un réactif en fin de réaction (sel de diazonium, par exemple) pour déceler l'amine libérée.

L'invention propose un nouveau type de substrats de peptidases, à base d'aminobenzène ou dérivés (composé I).

Ce dernier, au fur et à mesure qu'il est libéré dans le milieu réactionnel lors de l'hydrolyse enzymatique, forme un complexe fortement coloré (rouge à bleu. généralement) par couplage oxydatif avec l'α-naphtol ou un dérivé (compose II) présent dans ce milieu.

La réaction de couplage peut également être obtenue en fin de test si l'addition du composé II intervient au terme de la réaction enzymatique.

Un avantage important de l'invention consiste à associer deux substrats dans le milieu réactionnel, selon le procédé, l'un à base du composé 1 et l'autre à base du composé II.

On peut imaginer ainsi tous les types de combinaisons de détection simultanée de deux activités enzymatiques (par exemple, une peptidase et une osidase, une estérase et une osidase, deux osidases, etc). Dans ces cas, il y aura la formation du complexe coloré III uniquement si les deux substrats sont hydrolysés. Cette combinaison peut être très utile pour les tests de diagnostic qui demandent une grande spécificité, notamment lorsqu'il s'agit de caractériser des micro-organismes.

L'un des exemples cités illustre l'intérêt de cette détection simultanée de deux activités enzymatiques dans le domaine de l'identification des bactéries.

Parmi les autres avantages de l'invention, il est possible de donner une double fonctionnalité au composé I, II est en effet possible de greffer chimiquement sur le composé I, d'une part sur le groupement -NH₂, un acide aminé ou une chaîne d'acides aminés terminés ou non par un agent bloquant, et d'autre part sur R₁, s'il s'agit d'un groupement hydroxyl (-OH), un ose, un phosphate ou un acide gras. On obtient une molécule que l'on peut qualifier de double substrat qui, pour générer le composé I à l'état libre dans le milieu réactionnel doit être hydrolysé par deux activités enzymatiques distinctes, d'une part, une peptidase et, d'autre part, une osidase, phosphatase ou estérase, selon la nature du produit greffé sur R₁.

Ainsi, en présence du composé II, le complexe coloré III ne peut être obtenu que si les deux activités enzymatiques sont présentes. L'intérêt de tels substrats à double fonctionnalité réside dans leur très grande spécificité qui peut être mise à profit pour caractériser des micro-organismes, par exemple.

Dans le même sens, on peut concevoir selon l'invention un mélange d'un substrat à double fonctionnalité à base du composé I. tel que décrit ci-dessus, et un substrat à base du composé II pour la recherche d'une autre osidase, phosphatase ou estérase. Dans ce cas, la production du complexe coloré III nécessite la présence simultanée de trois activités enzymatiques, afin de libérer les composés I et II. Deux activités enzymatiques permettent d'hydrolyser la molécule à base du composé I et une activité enzymatique permet d'hydrolyser la molécule à base du composé II. On augmente ainsi encore la spécificité du test.

Selon l'invention, il est aussi possible de combiner différents types de substrats dans le même milieu réactionnel, en particulier des substrats connus dans l'art antérieur et des substrats selon le procédé revendiqué. Cela permet de mettre en oeuvre différentes réactions colorées, et donc de révéler en même temps plusieurs activités enzymatiques au sein du même milieu réactionnel.

L'invention peut s'appliquer à la recherche d'activités enzymatiques dans différents types d'échantillons, biologiques ou non.

Elle peut également être utilisée pour caractériser des micro-organismes comme des bactéries ou des levures. Les tests enzymatiques peuvent alors être pratiqués en milieu liquide dans des tubes individuels ou dans des supports compartimentés, comme les plaques de microtitration, par exemple. Ils peuvent être pratiqués également en milieu gélosé (dans des boîtes de Pétri, par exemple) avec coloration des colonies produisant les activités enzymatiques recherchées.

La présente invention concerne donc un procédé de détection d'au moins deux activités enzymatiques par l'intermédiaire de substrats, tels que décrits ci-dessus, qui consiste à :
- mettre les substrats en présence d'au moins un type de bactéries,
- attendre que les bactéries hydrolysent les substrats, et
- détecter les activités enzymatiques basée sur la formation d'un complexe coloré à partir du couplage oxydatif des deux parties marqueur.

Le couplage oxydatif est facilité soit par :
- l'ajout d'au moins un oxydant dans le milieu réactionnel,
- la production, lors d'un processus métabolique au sein de ce milieu réactionnel, d'au moins un oxydant,
- la présence d'oxygène endogène dans ledit milieu réactionnel.

Les exemples cités ci-dessous permettront de mieux comprendre l'invention. Ils concernent uniquement le domaine de la bactériologie, mais il est clair que le procédé peut s'appliquer à tous les autres domaines de l'enzymologie, impliquant la recherche d'hydrolases.

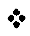 **Exemple 1 : Détection de la β-glucosidase en milieu liquide (méthode 1) :**

Le milieu réactionnel proposé dans cet exemple a pour formulation :
- peptone de viande 10 g
- NaCl 5 g
- α-naphtyl-β-D-glucopyranoside 0.5 g
- 4-aminophénol 50 mg
- H₂O 1000 ml
- pH 7.0

Ce milieu est stérilisé par filtration sur filtre Millipore 0,22 µ et réparti dans des tubes stériles à raison de 5 ml par tube.

Le test de recherche de la β-glucosidase est pratiqué sur 4 souches cultivées pendant 24H à 35-37°C sur gélose Trypticase-Soja. Ces souches appartiennent respectivement aux espèces :
- *Escherichia coli,*
- *Staphylococcus sciuri,*
- *Streptococcus pyogenes,*
- *Enterococcus faecalis.*

A partir des cultures précédentes, on réalise des suspensions aqueuses ajustées à 0,5 McFarland.

100 microlitres de ces suspensions sont utilisés pour ensemencer quatre tubes de milieu réactionnel, chaque tube correspondant à une souche.

Les tubes bouchés sont incubés à 35-37°C pendant 18 à 24H.

Après l'incubation, on observe l'apparition d'une coloration pourpre correspondant à la formation du complexe indophénol et traduisant la présence d'une activité β-glucosidase.

Les résultats obtenus sont les suivants :

| **souche** | **résultat théorique attendu** | **résultat obtenu** |
|---|---|---|
| *E. coli* | - | - |
| *S. sciuri* | + | + |
| *S. pyogenes* | - | - |
| *E. faecalis* | + | + |

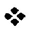 **Exemple 2 : Détection de la β-glucosidase en milieu liquide (méthode 2) :**

Le milieu réactionnel proposé dans cet exemple a pour formulation :
- peptone de viande 10 g
- NaCl 5 g
- α-naphtol 50 mg
- 4-aminophényl-β-D-glucopyranoside 0.5 g
- H₂O 1000 ml
- pH 7.0

La préparation du milieu, le choix des souches testées et la pratique du test sont identiques à la procédure décrite dans l'exemple 1.

L'apparition d'une coloration pourpre due à la formation du complexe indophénol indique la présence d'une activité β-glucosidase.

Les résultats obtenus sont identiques à ceux de l'exemple 1, à savoir :

| **souche** | **résultat théorique attendu** | **résultat obtenu** |
|---|---|---|
| *E. coli* | - | - |
| *S. sciuri* | + | + |
| *S. pyogenes* | - | - |
| *E. faecalis* | + | + |

Ces résultats montrent qu'il est possible de détecter une activité de type osidase en utilisant selon l'invention aussi bien un substrat à base du composé I qu'un substrat à base du composé II. Il en serait de même pour rechercher des activités de types estérases ou phosphatases.

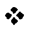 **Exemple 3 : Détection de la pyroglutamyl-aminopeptidase en milieu** liquide :

Le milieu réactionnel proposé dans cet exemple a pour formulation :
- peptone de viande 10 g
- NaCl 5 g
- α-naphtol 50 mg
- L-pyroglutamyl-4-amino-2,6-dichlorophénol 0.5 g
- H₂O 1000 ml
- pH 7.0

La préparation du milieu, le choix des souches testées et la pratique du test sont identiques à la procédure décrite dans l'exemple 1.

L'apparition d'une coloration bleue due à la formation du complexe indophénol met en évidence une activité pyroglutamyl-aminopeptidase. A noter que la teinte bleue résulte de la présence de groupements chlore en position 2 et 6 sur le 4-aminophénol.

Les résultats obtenus sont les suivants :

| **souche** | **résultat théorique attendu** | **résultat obtenu** |
|---|---|---|
| *E. coli* | - | - |
| *S. sciuri* | - | - |
| *S. pyogenes* | + | + |
| *E. faecalis* | + | + |

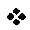 **Exemple 4 : Recherche combinée de deux activités enzymatiques selon le procédé de l'invention. Application à la détection simultanée de la β-glucosidase et de la pyroglutamyl-aminopeptidase :**

Le milieu réactionnel proposé dans cet exemple a pour formulation :
- peptone de viande 10 g
- NaCl 5 g
- α-naphtyl-b-D-glucopyranoside 0.5 g
- L-pyroglutamyl-4-amino-2,6-dichlorophénol 0.5 g
- H₂O 1000 ml
- pH 7.0

La préparation du milieu, le choix des souches testées et la pratique du test sont identiques à la procédure décrite dans l'exemple 1.

L'apparition d'une coloration bleue due à la formation du complexe indophénol n'est possible que si les deux substrats sont hydrolysés. Elle traduit donc une combinaison d'activités β-glucosidase et pyroglutamyl-aminopeptidase dans le même milieu réactionnel. L'absence de l'une des activités empêche la formation du complexe coloré.

Les résultats obtenus sont les suivants :

| **souche** | **résultat théorique attendu** | **résultat obtenu** |
|---|---|---|
| *E. coli* | - | - |
| *S. sciuri* | - | - |
| *S. pyogenes* | - | - |
| *E. faecalis* | + | + |

Cet exemple illustre clairement un des avantages de l'invention en permettant une plus grande sélectivité dans la caractérisation des microorganismes (*E. faecalis,* dans cette application).

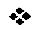 **Exemple 5 : Caractérisation de bactéries en milieu gélosé par recherche simultanée des activités β-galactosidase (βGAL) et L-alanine-aminopeptidase (Ala) :**

A un milieu trypticase soja gélosé, sont ajoutés de l'α-naphtyl-β-D-galactopyranoside et de la L-alanyl-N,N'-diméthyl-p-phénylène-diamine-naphtalène sulfonylhydrazide. Ce substrat est dérivé du composé I selon l'invention.

Le milieu est réparti et ensemencé de la façon suivante :
- six boîtes avec des cultures pures :
   - *Escherichia coli* [βGAL (+), Ala (+)]
   - *Klebsiella pneumoniae* [βGAL (+), Ala (+)]
   - *Salmonella typhimurium* [βGAL (-), Ala (+)]
   - *Pseudomonas aeruginosa* [βGAL (-), Ala (+)]
   - *Staphylococcus xylosus* [βGAL (+), Ala (-)]
   - *Candida albicans* [βGAL (-), Ala (-)].
- deux boîtes avec les mélanges de souches *E. coli* / *S. xylosus* et *S. typhimurium* / *C. albicans.*

Les milieux sont incubés à 35-37°C. Après 18 - 24H d'incubation, seules les boîtes contenant les souches d'*E*. *coli* et *K pneumoniae* [βGAL (+), Ala (+)] présentent des colonies colorées en bleu.

Avec le mélange *E. coli* / *S. xylosus,* seules les colonies d'*E*. *coli* sont bleues, celles de *S. xylosus* restant incolores.

Tout comme dans l'exemple 4, la production du complexe coloré III à base d'indophénol a donc nécessité la présence conjointe de deux activités enzymatiques, ici la β-galactosidase et la L-alanine-aminopeptidase.

En revanche, la présence de l'une seulement ou aucune de ces deux activités n'entraîne pas la formation du complexe coloré.

Comme cela est le cas dans l'exemple 5, le choix d'un dérivé approprié de la phénylène-diamine permet d'obtenir une coloration qui reste au voisinage immédiat des colonies et ainsi de distinguer deux populations différentes en mélange.

Cet exemple montre qu'il est possible, selon l'invention, de coloré spécifiquement des colonies à la surface d'un milieu gélosé et donc de les caractériser. Dans le cas présent, il pourrait s'appliquer à la détection des bactéries coliformes qui sont des bacilles à Gram négatif possédant les deux activités β-galactosidase et L-alanine-aminopeptidase.

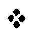 **Exemple 6 : Recherche combinée de trois activités enzymatiques selon le procédé de l'invention. Application à la détection simultanée de la β-galactosidase (βGAL), de la β-glucosidase (βGLU) et de la L-alanine-aminopeptidase (Ala):**

Le milieu réactionnel proposé dans cet exemple a pour formulation :
- peptone de viande 10 g
- NaCl 5 g
- α-naphtyl-β-D-glucopyranoside 0.5 g
- L-alanyl-4-amino-2,6-dichlorophényl-β -D-galactopyranoside 0.5 g
- H₂O 1000 ml
- pH 7.0

La préparation du milieu est identique à la procédure décrite dans l'exemple 1.

Le test de recherche des 3 activités enzymatiques est pratiqué sur 5 souches cultivées pendant 24H à 35-37°C sur gélose Trypticase-Soja. Ces souches appartiennent respectivement aux espèces :
- *Escherichia coli* [βGAL (+),βGLU (-), Ala (+)]
- *Klebsiella pneumoniae* [βGAL (+),βGLU (+), Ala (+)]
- *Salmonella typhimurium* [βGAL (-),βGLU (-), Ala (+)]
- *Stenotrophomonas maltophilia* [βGAL (-),βGLU (+), Ala (+)]
- *Staphylococcus saprophyticus* [βGAL (+),βGLU (-), Ala (-)]

L'apparition d'une coloration bleue due à la formation du complexe indophénol n'est possible que si les 3 substrats sont hydrolysés. Elle traduit donc une combinaison d'activités β-galactosidase, β-glucosidase et L-alanine-aminopeptidase dans le même milieu réactionnel, comme c'est le cas pour la souche de *K. pneumoniae.* L'absence de l'une des activités empêche la formation du complexe coloré.

Les résultats obtenus sont les suivants :

| **souche** | **résultat théorique attendu** | **résultat obtenu** |
|---|---|---|
| *E. coli* | - | - |
| *K. pneumoniae* | + | + |
| *S. typhimurium* | - | - |
| *S. maltophilia* | - | - |
| *S. sapropriticus* | - | - |

Cet exemple montre une nouvelle fois les potentialités de l'invention pour mettre en oeuvre des tests hautement sélectifs.

Les six exemples cités décrivent uniquement des tests enzymatiques selon l'invention.

Il est bien entendu possible de combiner ceux-ci aux tests connus dans l'art antérieur, utilisant des substrats chromogéniques ou fluorogéniques.

### II - Procédé et dispositif d'identification :

Le principe du milieu, utilisé avec le procédé et le dispositif d'identification, est basé sur l'utilisation des substrats jumelés décrits précédemment.

Selon un exemple représentatif, le procédé d'identification des GRAM utilise un substrat à base d'amino-benzène ou un de ses dérivés, tel que le DiMéthyl-paraPhénylène-Diamine (DMpPD). Dans l'exemple qui suit, le substrat est constitué par de l'Alanine-Diméthyl-paraPhénylène-Diamine (AlaDMpPD). L'activité mise en évidence est donc l'Alanine-aminopeptidase, activité spécifique des bactéries à Gram négatif.

Son fonctionnement est le suivant. Il y a, par hydrolyse enzymatique, libération du groupement DMpPD. Ensuite le DMpPD se lie avec l'α-naphtol (ou un dérivé) par couplage oxydatif facilité par un oxydant, le ferricyanate de potassium (K₃Fe(CN)₆). Il y a alors formation d'un complexe coloré gris violet.

Dans un premier temps, le dispositif est défini de la manière suivante :
- un milieu biogélytone contenant comme substrat : l'AlaDMpPD, et
- un écouvillon imprégné d'un mélange réactionnel constitué d'α-naphtol et de ferricyanate de potassium.

Un autre dispositif a été défini qui est encore plus spécifique dans l'identification du Gram et est également utilisable avec les géloses au sang de type Columbia. II consiste en :
- un milieu constitué d'une gélose au sang de type Columbia (avec ou sans AlaDMpPD), et
- un mélange réactionnel qui imprègne un écouvillon et qui est constitué à base d'α-naphtol, de ferrycyanate de potassium et d'AlaDMpPD puis séché.

Ce mélange réactionnel contenu par l'écouvillon, a été encore amélioré par l'ajout d'une étape de séchage des écouvillons, ce qui facilite leur manipulation ultérieure.

La composition exacte est la suivante :
- pour la gélose : présence ou non d'AlaDMpPD à 0. 075 gramme par litre (g/l), et
- pour l'écouvillon :
   - α-naphtol de 0,01 à 5 g/l, préférentiellement de 0,1 à 1 g/l, et par exemple de 0,5 g/l,
   - ferricyanate de potassium de 0.01 à 5 g/l, préférentiellement de 0.1 à 1 g/l, et par exemple de 0,5 g/l, et
   - AlaDMpPD de 0,01 à 5 g/l, préférentiellement de 0.1 à 1 g/l, et par exemple de 0,35 g/l.

Les résultats obtenus en terme de sensibilité et spécificité sont alors les meilleurs obtenus. Dans ce cas, la détection de la réaction n'est pas instantanée. II faut attendre 15 à 30 secondes (s) pour observer l'apparition d'une éventuelle coloration, temps nécessaire à l'hydrolyse du substrat par l'aminopeptidase bactérienne:

Soixante et une souches sur différents milieux tels que : gélose chocolat Polyvitex (marque déposée), milieu CPS ID2 (marque déposée), TSA +/- sang et Columbia +/- sang, ont été testées avec des écouvillons imprégnés dα-naphtol, de ferrycyanate de potassium et d'AlaDMpPD puis séchés. Cette étude a montré que ce système était compatible avec la majorité des milieux conventionnels et chromogéniques usuels.

Pour améliorer encore le temps de réaction, un activateur de la réaction est incorporé dans le milieu.

Dans ce contexte d'activation de la réaction, il s'est avéré que l'ajout dans le milieu d'une faible quantité d'AlaDMpPD et/ou d'α-naphtol permet la détection de la réaction sans diminuer la sensibilité. De plus, le temps de détection est ainsi considérablement diminué, puisqu'il est porté de 0 à 10 s. Cette faible quantité correspond à une concentration de 0,01 à 0,5 g/l, préférentiellement de 0,05 à 0,1 g/l, et par exemple de 0,075 g/l.

Selon une variante de réalisation, il est également possible d'ajouter d'un liant sur l'écouvillon. L'apport d'un liant permet à la fois de resserrer les fibres de la tête de l'écouvillon et de limiter le relargage de produits présents sur l'écouvillon dans la suspension de l'inoculum. Un tel liant peut être constitué par la PolyVinyl Pyrolidone (PVP), qui permet en effet non seulement de répondre aux critères précédents mais aussi d'augmenter sensiblement la spécificité. Dans la composition précédemment décrite avec les concentrations, le PVP est introduit dans les constituants de la tête viscose de l'écouvillon dans une concentration de 1 à 50 g/l, préférentiellement de 10 à 25 g/l, et par exemple de 15 g/l.

### 1°) Expérimentation :

Une évaluation de ce milieu a donc été réalisée sur cent trente trois (133) souches réparties selon les espèces suivantes. Le tableau 1, qui suit, énumère les espèces testées ainsi que le nombre de souche pour chaque espèce.

**Tableau 1 : Espèces testées et nombre de souches pour chaque espèce**

| **Espèce** | **Nombre de souches** |
|---|---|
| *Acinetobacter baumanii* | 2 |
| *Acinetobacter junii* | 2 |
| *Citrobacter freundii* | 3 |
| *Citrobacter spp* | 1 |
| *Enterobacter cloacae* | 3 |
| *Enterobacter intermidis* | 1 |
| *Enterobacter spp* | 1 |
| *Escherichia coli* | 4 |
| *Klebsiella oxytoca* | 5 |
| *Klebsiella pneumoniae* | 4 |
| *Morganella morganii* | 4 |
| *Proteus mirabilis* | 5 |
| *Proteus vulgaris* | 5 |
| *Providencia stuartii* | 4 |
| *Pseudomonas aeruginosa* | 4 |
| *Pseudomonas fluorescens* | 5 |
| *Serratia marcescens* | 4 |
| *Bacillus cereus* | 4 |
| *Bacillus lentus* | 1 |
| *Bacillus mycoïdes* | 2 |
| *Bacillus subtilis* | 2 |
| *Corynebacterium aquaticum* | 2 |
| *Corynebacterium ulcerans* | 2 |
| *Enterococcus faecalis* | 5 |
| *Enterococcus faecium* | 3 |
| *Enterococcus gallinarum* | 3 |
| *Haemophilus haemolyticus* | 2 |
| *Haemophilus influenzae* | 2 |
| *Lactobacillus casei* | 4 |
| *Listeria monocytogenes* | 4 |
| *Staphylococcus aureus* | 3 |
| *Staphylococcus epidermidis* | 3 |
| *Staphylococcus haemolyticus* | 3 |
| *Staphylococcus saprophyticus* | 3 |
| *Streptococcus agalactiae* | 4 |
| *Streptococcus pneumoniae* | 3 |
| *Streptococcus pyogenes* | 3 |
| *Candida albicans* | 3 |
| *Candida glabrata* | 3 |
| *Candida guilliermondii* | 1 |
| *Candida kefyr* | 1 |
| *Candida krusei* | 3 |
| *Candida parapsilosis* | 1 |
| *Candida tropicalis* | 4 |
| *Trichosporon asteroïdes* | 1 |
| *Trichosporon mucoïdes* | 1 |
| TOTAL | 133 |

Le tableau 2 énumère les résultats obtenus :

**Tableau 2 : Sensibilité et spécificité des espèces testées**

| | |
|---|---|
| Sensibilité | 93 % |
| Spécificité vis-à-vis des Gram + | 81 à 90 % |
| Spécificité vis-à-vis des levures | 89 à 100 % |

Parmi les résultats obtenus sur les souches testées, certaines souches sont des faux négatifs. Ces faux négatifs sont deux (2) des quatre (4) souches de *P. aeruginosa,* deux (2) des cinq (5) souches de *P. fluorescens,* et une (1) des deux (2) souches d'*H. influenzae,* Certaines souches sont des faux positifs. Ces faux positifs sont constitués par les deux (2) souches de *C*. *ulcerans,* une (1) des cinq (5) souches d'*E*. *faecalis,* les trois (3) souches de *S. pyogenes,* deux (2) des quatre (4) souches de *S. agalactiae,* la souche de *C. guilliermondii* et la souche de *C. parapsilosis.*

### 2°) Lisibilité du test :

La quasi-totalité des bactéries à Gram négatif, à l'exception des *Pseudomonas,* donnent une coloration gris violette franche dont l'intensité est supérieure strictement à 2 (sur une échelle semi-quantitative allant jusqu'à 4), tandis que les bactéries à Gram positif ou qui sont faux-positifs, à l'exception de *S. pyogenes,* ont une intensité de coloration inférieure à 1 sur cette même échelle.

### 3°) Temps de détection :

Environ 90% des bactéries à Gram négatif sont détectées entre 0 et 10 s. la plupart des bactéries à Gram positif ou qui sont faux-positifs, sont détectées après 10 s, à l'exception des souches de *S. pyogenes.*

Le milieu actuel présente donc une bonne sensibilité et une bonne spécificité. Les autres souches faux-positifs sont généralement d'intensité très faible et présentent un temps de détection supérieur à 10 s. Ces souches sont donc plutôt des souches douteuses plutôt que des faux-positifs.

### 4°) Définition de l'écouvillon :

Le modèle d'écouvillon, particulièrement intéressant, est un écouvillon qui répond aux critères suivants :
- un bâtonnet plastique de diamètre 2,5 millimètres (mm) et de longueur 150 mm, et
- avec une tête viscose de diamètre inférieur ou égal à 4,5 mm.

### 5°) Mode de fabrication et de manipulation des écouvillons :

Les conditions de fabrication sont les suivantes :
- imbibition dans une solution telle que définie ci-dessus pendant 2 à 3 minutes (mn),
- séchage pendant 2 heures (h) à 37°C, et
- conditionnement en sac plastique étanche et à l'abri de la lumière.

### 6°) Conditionnement des écouvillons:

Les écouvillons peuvent par exemple être stockés de manière stérile (rayons gamma) dans un emballage contenant un (1), vingt-cinq (25) ou cent (100) à mille (1000) de ces écouvillons. Néanmoins, un conditionnement de vingt (20) à vingt-cinq (25) écouvillons pas obligatoirement stérilisés après séchage est tout à fait envisageable. Le conditionnement s'effectue avec ou sans substance de dessiccation, et à l'abri de la lumière.

### 7°) Paramètres critiques :

L'utilisation des substrats jumelés est donc identique à celle qui est exposée dans le début de la description. Ainsi, la tête de l'écouvillon contient au moins deux molécules. Ainsi dans l'exemple donné, une première molécule (AlaDMpPD) est constituée d'une partie marqueur (DMpPD) non chromogène associée à au moins une partie spécifique (alanine) cible de l'enzyme (Alanine-aminopeptidase) et une seconde molécule constituée d'une autre substance non chromogène (α-naphtol), la partie marqueur non chromogène (AlaDMpPD), une fois libérée, réagit avec la seconde molécule (α-naphtol) pour former une molécule chromogène.

L'utilisation de l'écouvillon sert de support de réactifs et permet la réalisation de la suspension de l'inoculum, qui sera utilisé dans d'autres systèmes d'identification et antibiogramme, sur la base des mêmes souches qui ont été évaluées précédemment. Ceci diminue donc considérablement les erreurs lors de repiquage de nouveaux micro-organismes « semblables » pour effectuer les opérations suivantes d'identification et d'antibiogramme, etc.

De plus, il est également possible de réaliser des écouvillons pour d'autres types de tests, par exemple oxydase, indole, estérase, phosphatase...

## Revendications

1. Composition comprenant deux substrats permettant de détecter la présence de l'activité enzymatique d'au moins deux enzymes, **caractérisée par le fait qu'**elle est constituée d'au moins deux molécules, une première molécule constituée d'une partie marqueur non chromogène associée à au moins une partie spécifique cible de la première enzyme et une seconde molécule constituée d'une autre partie marqueur non chromogène associée à une partie spécifique cible de la seconde enzyme, et que les parties marqueur non chromogènes, une fois libérées, réagissent pour former une molécule chromogène,
la partie marqueur non chromogène de la première molécule étant constituée d'amino-benzène ou l'un de ses dérivés, 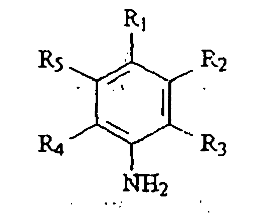 la partie marqueur non chromogène de la seconde molécule étant constituée d'α-naphtol ou l'un de ses dérivés : 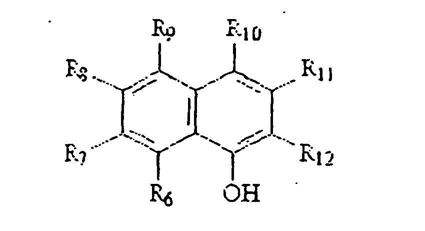 et la molécule chromogène obtenue étant constituée par : 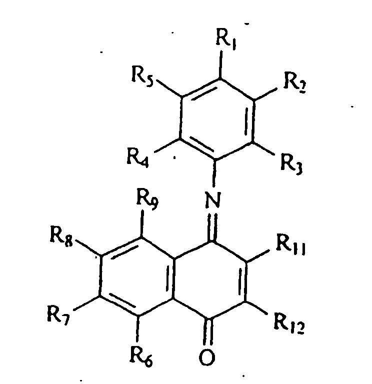 formules dans lesquelles :
le radical R₁ est constitué de -OH, -SH ou -NXZ,
le radical R₁₀ est choisi parmi -H, -Br, -CI, -I, et les groupes d'atomes enlevés durant le couplage oxydatif,
chaque radical R₂ à R₉, R₁₁ ou R₁₂ est choisi parmi -H, -OH, -Br, -Cl, -I, -CH₃, CH₂-CH₃, -OCH₃, -OCH₂CH₃, -COOH, et
X et/ou Z est constitué de -H, -CH₃, -CH₂CH₃,

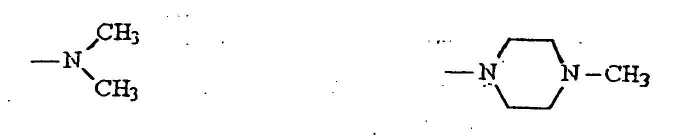

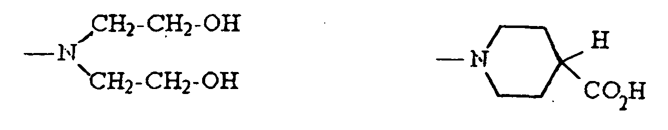

2. Composition selon la revendication 1, **caractérisée en ce que** R₁₀ représente SH.

3. Composition selon la revendication 1 ou 2, **caractérisée par le fait qu**'au moins l'un des couples de radicaux R₂/R₃ et R₄/R₅ est constitué d'un système aromatique, alicyclique ou hétérocyclique.

4. Procédé de détection d'au moins deux activités enzymatiques par l'intermédiaire de substrats, selon l'une quelconque des revendications 1 à 3, **caractérisé en ce qu'**il consiste à
- mettre les molécules constituant au moins deux substrats en présence d'au moins un type de bactéries contenu dans un échantillon à tester,
- attendre que les bactéries hydrolysent les substrats, et
- détecter les activités enzymatiques basées sur la formation d'un complexe coloré à partir du couplage oxydatif des deux parties marqueur.

5. Procédé, selon la revendication 4, **caractérisé en ce que** les molécules sont présentes dans un matériau absorbant et sont mises en contact avec l'échantillon, et que après détection, les bactéries ainsi prélevées sont remises en suspension afin de permettre des analyses ultérieures (identifications, antibiogrammes, etc).

6. Procédé, selon la revendication 4 ou 5, **caractérisé en ce que** la composition réactionnelle du substrat, éventuellement contenue dans le matériau absorbant, comprend un oxydant.

7. Procédé selon la revendication 6, **caractérisé en ce que** l'oxydant est le ferricyanate de potassium.

8. Procédé, selon la revendication 6, **caractérisé en ce que** la composition réactionnelle comprend un activateur de la réaction, tel qu'une faible quantité de la première molécule et/ou de la deuxième molécule, présente (s) dans l'échantillon à tester.

9. Procédé, selon l'une quelconque des revendications 6 à 8, **caractérisé en ce que** la composition comprend un liant.

10. Procédé selon la revendication 9, **caractérisé en ce que** le liant le Poly Vinyl Pyrrolidone (PVP).

11. Procédé, selon l'une quelconque des revendications 6 à 10, pour identifier le Gram d'une espèce bactérienne à tester, **caractérisé en ce que** la première molécule est constituée par de l'AlaDMpPD, et que la seconde molécule est constituée par de l'α-naphtol.

12. Procédé, selon l'une quelconque des revendications 6 à 11, **caractérisé en ce que** la composition du matériau absorbant est la suivante:
- α-naphtol de 0,01 à 5g/l, préférentiellement de 0,1 à 1 g/l,
- ferricyanate de potassium de 0,01 à 5 g/l, préférentiellement de 0,1 à 1 g/l, et
- AlaDMpPD de 0,01 à 5 g/l, préférentiellement de 0,1 à 1 g/l,.

13. Procédé selon la revendication 12, **caractérisé en ce que** le matériau absorbant contient 0,5 g/l d'α-naphtol.

14. Procédé selon la revendication 13 ou 14, **caractérisé en ce que** le matériau absorbant contient 0,5 g/l de ferricyanate de potassium.

15. Procédé selon l'une quelconque des revendications 12 à 14, **caractérisé en ce que** le matériau absorbant contient 0,35 g/l de AlaDMpPD.

16. Procédé, selon la revendication 8, **caractérisé en ce que** l'activateur est constitué par de l'AlaDMpPD à une concentration de 0,01 à 0,5 g/l, préférentiellement de 0,05 à 0,1 g/l.

17. Procédé selon la revendication 16, **caractérisé en ce que** la concentration de l'AlaDMpPD est de 0,075 g/l.

18. Procédé, selon la revendication 9 ou 10, **caractérisé en ce que** la composition comprend en outre de 1 à 50 g/l, préférentiellement de 10 à 25 g/l.

19. Procédé selon la revendication 18, **caractérisé en ce que** la composition comprend 15 g/l de PVP.

20. Utilisation de substrat, selon l'une quelconque des revendications 1 a 3, pour la détection d'une activité enzymatique de type peptidase.

21. Dispositif pour la détection, dans un échantillon, d'au moins deux activités enzymatiques, **caractérisé en ce qu'**il comporte un support inerte vis à vis du matériau absorbant et des substrats qu'il contient et de l'échantillon testé, une tête en matériau absorbant montée sur ce support et une composition selon l'une quelconque des revendications 1 à 3.

22. Dispositif selon la revendication 21, **caractérisé en ce que** le support est en plastique et le matériau absorbant est du viscose.

## Claims

1. Composition comprising two substrates for detecting the presence of the enzymatic activity of at least two enzymes, **characterized in that** it consists of at least two molecules, a first molecule consisting of a non-chromogenic labelling portion associated with at least one specific target portion of the first enzyme and a second molecule consisting of another non-chromogenic labelling portion associated with a specific target portion of the second enzyme, and **in that** the non-chromogenic labelling portions, once released, react to form a chromogenic molecule, the non-chromogenic labelling portion of the first molecule consisting of aminobenzene or a derivative thereof: 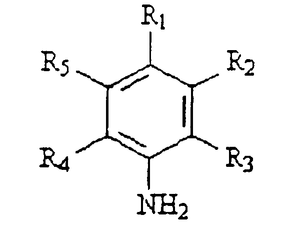 the non-chromogenic labelling portion of the second molecule consisting of α-naphthol or a derivative thereof: 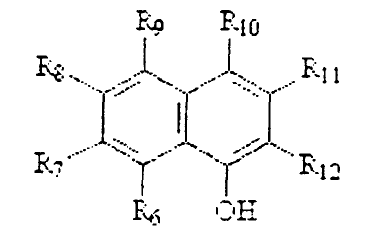 and the chromogenic molecule obtained consisting of: 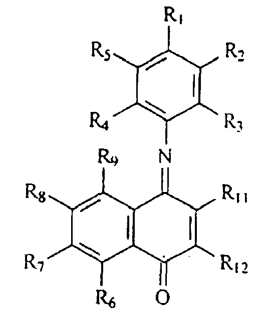 formulae in which:
the radical R₁ consists of -OH, -SH or -NXZ, the radical R₁₀ is chosen from among -H, -Br, -Cl or -I, and
the groups of atoms removed during the oxidative coupling,
each radical R₂ to R₉, R₁₁ or R₁₂ is chosen from among -H, -OH, -Br, -Cl, -I, -CH₃, -CH₂CH₃, -OCH₃, -OCH₂CH₃ or -COOH, and
X and/or Z consists of -H, -CH₃, -CH₂CH₃,

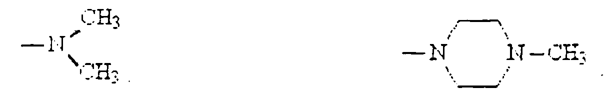

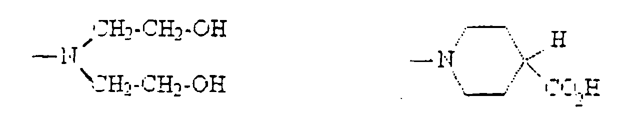

2. Composition according to Claim 1, **characterized in that** R₁₀ represents SH.

3. Composition according to Claim 1 or 2, **characterized in that** at least one of the pairs of radicals R₂/R₃ and R₄/R₅ consists of an aromatic, alicyclic or heterocyclic system.

4. Process for detecting at least two enzymatic activities via substrates, according to any one of Claims 1 to 3, **characterized in that** it consists in:
- placing the molecules consisting of at least two substrates in the presence of at least one type of bacteria, which is contained in a test sample,
- waiting for the bacteria to hydrolyse the substrates, and
- detecting the enzymatic activities based on the formation of a coloured complex from the oxidative coupling of the two labelling portions.

5. Process, according to Claim 4, **characterized in that** the molecules are present in an absorbent material and are placed in contact with the sample, and **in that**, after detection, the bacteria thus withdrawn are resuspended in order to allow subsequent analyses (identifications, antibiotic assays, etc.).

6. Process, according to Claim 4 or 5, **characterized in that** the reaction composition of the substrate, optionally contained in the absorbent material, comprises an oxidizing agent.

7. Process according to Claim 6, **characterized in that** the oxidizing agent is potassium ferricyanide.

8. Process, according to Claim 6, **characterized in that** the reaction composition comprises a reaction activator, such as a small amount of the first molecule and/or of the second molecule, which is (are) present in the sample to be tested.

9. Process, according to any one of Claims 6 to 8, **characterized in that** the composition comprises a binder.

10. Process according to Claim 9, **characterized in that** the binder is PolyVinylPyrrolidone (PVP).

11. Process, according to any one of Claims 6 to 10 for the Gram identification of a bacterial species to be tested, **characterized in that** the first molecule consists of AlaDMpPD, and **in that** the second molecule consists of α-naphthol.

12. Process, according to any one of Claims 6 to 11, **characterized in that** the composition of the absorbent material is as follows:
- α-naphthol from 0.01 g/l to 5 g/l, preferably from 0.1 g/l to 1 g/l,
- potassium ferricyanide from 0.01 g/l to 5 g/l, preferably from 0.1 g/l to 1 g/l, and
- AlaDMpPD from 0.01 g/l to 5 g/l, preferably from 0.1 g/l to 1 g/l.

13. Process according to Claim 12, **characterized in that** the absorbent material contains 0.5 g/l of α-naphthol.

14. Process according to Claim 13 or 14, **characterized in that** the absorbent material contains 0.5 g/l of potassium ferricyanide.

15. Process according to any one of Claims 12 to 14, **characterized in that** the absorbent material contains 0.35 g/l of AlaDMpPD.

16. Process, according to Claim 8, **characterized in that** the activator consists of AlaDMpPD at a concentration of from 0.01 g/l to 0.5 g/l, preferably from 0.05 g/l to 0.1 g/l.

17. Process according to Claim 16, **characterized in that** the concentration of AlaDMpPD is 0.075 g/l.

18. Process, according to Claim 9 or 10, **characterized in that** the composition also comprises from 1 g/l to 50 g/l, preferably from 10 g/l to 25 g/l.

19. Process, according to Claim 18, **characterized in that** the composition comprises 15 g/l of PVP.

20. Use of a substrate, according to any one of Claims 1 to 3, for detecting an enzymatic activity of peptidase type.

21. Device for detecting, in a sample, at least two enzymatic activities, **characterized in that** it comprises a support which is inert with respect to the absorbent material, to the substrates it contains and to the test sample, a head of absorbent material mounted on this support and a composition according to any one of Claims 1 to 3.

22. Device according to Claim 21, **characterized in that** the support is made of plastic and the absorbent material is of viscose.

## Patentansprüche

1. Zwei Substrate umfassende Zusammensetzung, die ermöglicht, das Vorhandensein der Enzymaktivität von mindestens zwei Enzymen nachzuweisen, **dadurch gekennzeichnet, dass** sie aus mindestens zwei Molekülen gebildet ist, einem ersten Molekül, das aus einem nicht chromogenen Marker-Bestandteil, der mit mindestens einem spezifischen Bestandteil, der ein Angriffsziel für das erste Enzym darstellt, assoziiert ist, aufgebaut ist, und einem zweiten Molekül, das aus einem weiteren nicht chromogenen Marker-Bestandteil, der mit einem spezifischen Bestandteil, der ein Angriffsziel für das zweite Enzym darstellt, aufgebaut ist, und **dadurch**, dass die nicht chromogenen Marker-Bestandteile, wenn sie freigesetzt sind, reagieren, um ein chromogenes Molekül zu bilden,
wobei der nicht chromogene Marker-Bestandteil des ersten Moleküls aus Aminobenzol oder einem seiner Derivate gebildet ist: 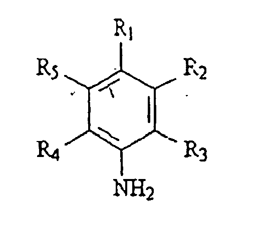 wobei der nicht chromogene Marker-Bestandteil des zweiten Moleküls aus α-Naphthol oder einem seiner Derivate gebildet ist: 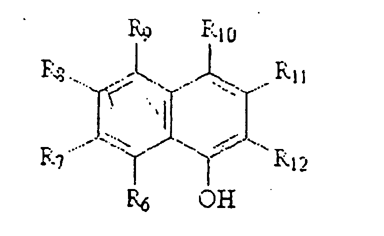 und das erhaltene chromogene Molekül aus 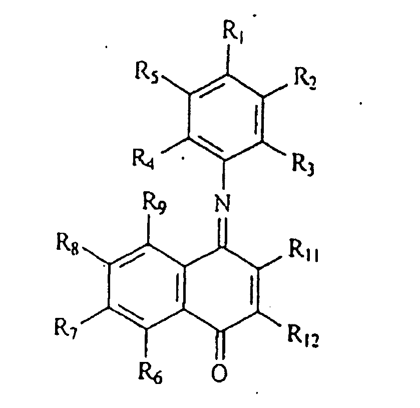 gebildet ist,
wobei in den Formeln:
das Radikal R₁ aus -OH, -SH oder -NXZ gebildet ist,
das Radikal R₁₀ aus -H, -Br, -Cl, -I und den bei der oxidativen Kopplung abgelösten Atomgruppen gewählt ist,
jedes Radikal R₂ bis R₉, R₁₁ oder R₁₂ aus -H, -OH, -Br, -Cl, -I, -CH₃, CH₂-CH₃, -OCH₃, -OCH₂CH₃, -COOH gewählt ist, und
X und/oder Z aus -H, -CH₃, -CH₂CH₃ 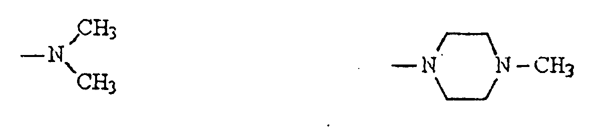 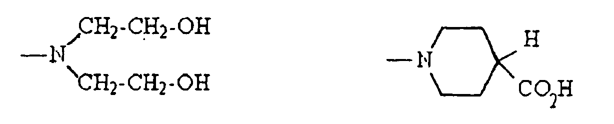 gebildet ist.

2. Zusammensetzung nach Anspruch 1, **dadurch gekennzeichnet, dass** R₁₀ SH repräsentiert.

3. Zusammensetzung nach Anspruch 1 oder 2, **dadurch gekennzeichnet, dass** mindestens eines der Radikalenpaare R₂/R₃ und R₄/R₅ aus einem aromatischen, alicyclischen oder heterocyclischen System gebildet ist.

4. Verfahren zum Nachweisen von mindestens zwei Enzymaktivitäten mittels Substraten nach einem der Ansprüche 1 bis 3, **dadurch gekennzeichnet, dass** es umfasst:
- Bringen von Molekülen, die mindestens zwei Substrate bilden, in Gegenwart mindestens einer Bakterienart, die in einer zu untersuchenden Probe enthalten ist;
- Warten, bis die Bakterien die Substrate hydrolysieren, und
- Nachweisen der Enzymaktivitäten basierend auf der Bildung eines Farbkomplexes durch oxidative Kopplung der zwei Marker-Bestandteile.

5. Verfahren nach Anspruch 4, **dadurch gekennzeichnet, dass** die Moleküle in einem absorbierenden Stoff vorliegen und mit der Probe in Kontakt gebracht sind, und dass nach dem Nachweis die folglich entnommenen Bakterien wieder in Suspension gebracht werden, um weitere Analysen (Identifizierungen, Antibiogramme usw.) zu ermöglichen.

6. Verfahren nach Anspruch 4 oder 5, **dadurch gekennzeichnet, dass** die reaktive Zusammensetzung des Substrats, die gegebenenfalls in dem absorbierenden Stoff enthalten ist, ein Oxidationsmittel umfasst.

7. Verfahren nach Anspruch 6, **dadurch gekennzeichnet, dass** das Oxidationsmittel Kaliumhexacyanoferrat(III) ist.

8. Verfahren nach Anspruch 6, **dadurch gekennzeichnet, dass** die reaktive Zusammensetzung ein Reaktionsaktivierungsmittel umfasst, wie etwa eine geringe Menge des ersten Moleküls und/oder des zweiten Moleküls, die in der zu untersuchenden Probe vorliegt.

9. Verfahren nach einem der Ansprüche 6 bis 8, **dadurch gekennzeichnet, dass** die Zusammensetzung ein Bindemittel umfasst.

10. Verfahren nach Anspruch 9, **dadurch gekennzeichnet, dass** das Bindemittel Polyvinylpyrrolidon (PVP) ist.

11. Verfahren nach einem der Ansprüche 6 bis 10, um die Gramfärbung einer Bakterienart, auf die untersucht wird, zu bestimmen, **dadurch gekennzeichnet, dass** das erste Molekül aus AlaDMpPD gebildet ist und das zweite Molekül aus α-Naphthol gebildet ist.

12. Verfahren nach einem der Ansprüche 6 bis 11, **dadurch gekennzeichnet, dass** die Zusammensetzung des absorbierenden Stoffes wie folgt ist:
- 0,01 bis 5 g/l, vorzugsweise 0,1 bis 1 g/l, α-Naphthol,
- 0,01 bis 5 g/l, vorzugsweise 0,1 bis 1 g/l, Kaliumhexacyanoferrat(III),
- 0,01 bis 5 g/l, vorzugsweise 0,1 bis 1 g/l, AlaDMpPD.

13. Verfahren nach Anspruch 12, **dadurch gekennzeichnet, dass** der absorbierende Stoff 0,5 g/l α-Naphthol enthält.

14. Verfahren nach Anspruch 13 oder 14, **dadurch gekennzeichnet, dass** der absorbierende Stoff 0,5 g/l Kaliumhexacyanoferrat(III) enthält.

15. Verfahren nach einem der Ansprüche 12 bis 14, **dadurch gekennzeichnet, dass** der absorbierende Stoff 0,35 g/l AlaDMpPD enthält.

16. Verfahren nach Anspruch 8, **dadurch gekennzeichnet, dass** das Aktivierungsmittel aus AlaDMpPD mit einer Konzentration von 0,01 bis 0,5 g/l, vorzugsweise von 0,05 bis 0,1 g/l, besteht.

17. Verfahren nach Anspruch 16, **dadurch gekennzeichnet, dass** die Konzentration des AlaDMpPD etwa 0,075 g/l beträgt.

18. Verfahren nach Anspruch 9 oder 10, **dadurch gekennzeichnet, dass** die Zusammensetzung außerdem 1 bis 50 g/l, vorzugsweise 10 bis 25 g/l umfasst.

19. Verfahren nach Anspruch 18, **dadurch gekennzeichnet, dass** die Zusammensetzung 15 g/l PVP umfasst.

20. Verwendung des Substrats nach einem der Ansprüche 1 bis 3 zum Nachweis einer Enzymaktivität vom Typ Peptidase.

21. Vorrichtung zum Nachweisen von mindestens zwei Enzymaktivitäten in einer Probe, **dadurch gekennzeichnet, dass** sie einen gegenüber dem absorbierenden Stoff und den Substraten, die er enthält, und der zu untersuchende Probe indifferenten Träger, einen Kopf aus absorbierendem Stoff, der an diesem Träger angebracht ist, und eine Zusammensetzung nach einem der Ansprüche 1 bis 3 umfasst.

22. Vorrichtung nach Anspruch 21, **dadurch gekennzeichnet, dass** der Träger aus Kunststoff ist und der absorbierende Stoff Viskose ist.
